# EUROPEAN PATENT APPLICATION

(11) **EP 1 889 634 A1**
(43) Date of publication of application: **20.02.2008**
(21) Application number: 06425592.0
(22) Date of filing: 18.08.2006
(51) Int. Cl.: A61M 1/10

(54) **A ventricular assist device and related computer program product**

(71) Applicant: NewCorTec S.p.A., 06034 Foligno (PG) (IT)
(72) Inventor: Rinaldi, Stefano, 06034 Foligno (Perugia) (IT); Zagara, Maurizio, 06034 Foligno (Perugia) (IT); Fierli, Manlio, 06034 Foligno (Perugia) (IT); Pesce Delfino, Gloria, 06034 Foligno (Perugia) (IT); Ercolani, Mauro, 06034 Foligno (Perugia) (IT)
(74) Representative: Bosotti, Luciano

(57) **Abstract**

A cardiac ventricular assist device or VAD (1) comprising a pumping unit (3-6) with a variable-volume chamber (3) capable of receiving a mass of blood, as well as a control unit (8) to control the pumping unit (3-6) to obtain contraction of the variable-volume chamber (3), with consequent expulsion of the blood collected in the chamber (3), and expansion of the variable-volume chamber (3) consequent on the flow of blood into the chamber (3). Likewise present are a flow line (7), such as a transcutaneous line, for a gaseous flow caused by the contraction and expansion of the variable-volume chamber (3). A sensor (10) sensitive to the gaseous flow in the flow line (7) generates a flow-meter signal that is indicative of the expulsion and inflow of blood with regard to the variable-volume chamber (3). The control unit (8) is sensitive to said flow-meter signal and utilises that signal to control the pumping unit (3-6), for example to cause the pumping unit (3-6) to operate in a condition of synchronous counterpulsation with regard to the natural heart, that is with the variable-volume chamber (3) capable of receiving blood from the assisted heart (C) when it is in a systolic phase and the variable-volume chamber (3) that expels the blood collected in the chamber (3), when the assisted heart (C) is in a diastolic phase.

## Description

### Field of the invention

The present invention relates, in general, to ventricular assist devices, currently known as VADs.

### Description of the related art

Ventricular assist devices (VADs) are essentially blood pumps designed to assist the left ventricle of a diseased heart to pump blood and to ensure an adequate degree of systemic perfusion to the patient's organs. These pumps take the blood from the heart, usually from the left ventricle, through an opening provided in an apical position, and pump it into the aorta. In this way they perform the work normally done by the left ventricle of the heart.

VADs may be subdivided into two categories depending on the flow that they produce: pulsatile and continuous. The pumping element of the pulsatile VAD is a chamber, comprising for example a flexible sac, thus a chamber with variable volume that cyclically fills with blood from the heart and then, being compressed by a special system (actuator), empties, sending a charge of blood into the aorta: these VADs thus operate like a natural heart ventricle and generate a pulsating flow of the physiological type. Continuous flow VADs comprise rotary pumps, for example centrifugal or axial (of the turbine type) and generate an almost continuous flow in the aorta.

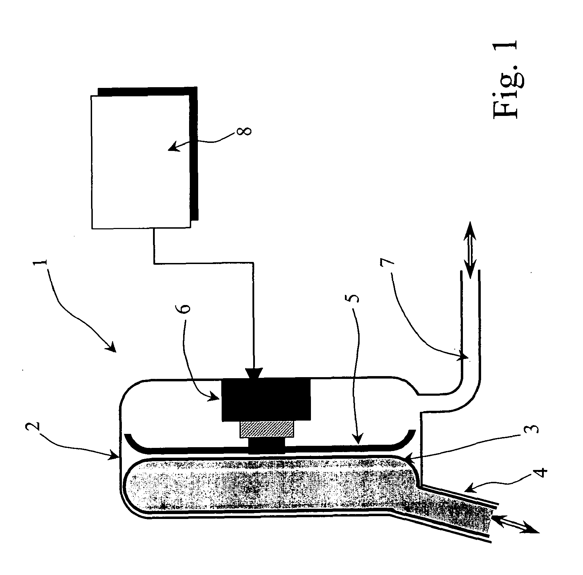

The present invention concerns, in general, VADs of the pulsatile type, for example of the type represented in diagram form in figure 1.

In figure 1, number 1 indicates a pumping device made, for example, following the criteria illustrated in EP-A-0 728 488 and EP-A-0 728 489.

The device 1 essentially comprises a casing 2 (made of substantially rigid and biocompatible material such as, for example, titanium) capable of being implanted in a thoracic or abdominal position. Inside the casing 2 there is a deformable sac 3, for example of polyurethane or of a silcon elastomer, capable of comprising a pumping chamber with flexible variable volume provided with respective conduits for the influx/ discharge of blood, indicated overall with 4.

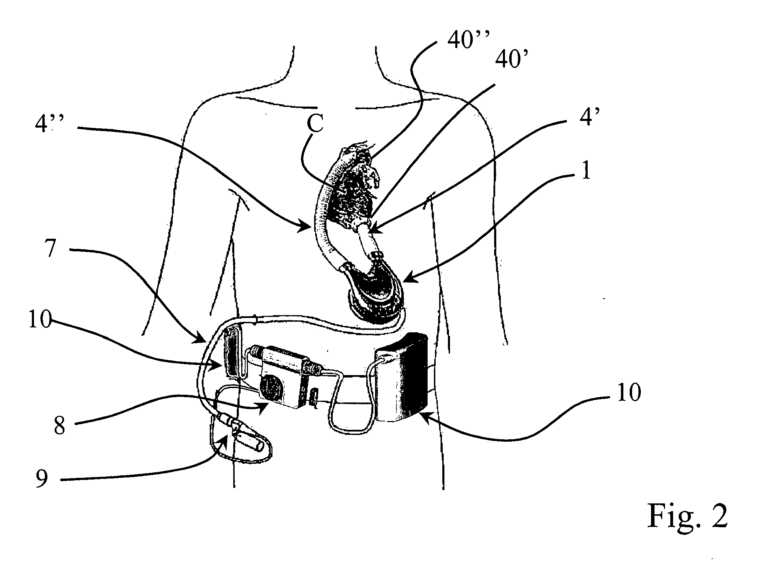

Thus, as may be inferred both from the description of the European patient applications mentioned above and from figure 2, which illustrates a typical implantation position of a ventricular assist device, in general two conduits 4', 4" are present, destined to act -- respectively -- as conduit for the intake or inflow (conduit 4') of blood into the sac 3 and as conduit for the discharge or outflow (conduit 4") of blood from the sac 3.

In the typical condition of implantation of the device 1, illustrated in figure 2, the conduits 4', 4" are connected -- through respective cannulas -- one to an opening 40' to withdraw blood, practised (generally in an apical position) in the left ventricle of the heart C of the wearer and the other to the aorta 40" of the wearer.

Although this is not immediately understandable from the drawings, the conduits bear associated to them respective valves (generally comprising cardiac valves of the prosthetic type, for example of the tilting disc type) destined to give the conduits 4', 4" the necessary unidirectional characteristic with regard to the blood flow from the left ventricle towards the sac 3 and from the sac 3 towards the aorta.

This connection enables the sac 3 to replace the left ventricle of the wearer in the work of pumping the blood towards the aorta, thus towards the circulatory system. For this purpose, the device 1 comprises a pumping unit including, alongside the sac and the valved conduits already described, an actuator consisting of elements 5 and 6 described in greater detail below and destined to enable the sac 3, alternately, to:
-- expand, so as to receive blood from the left ventricle of the wearer, that in this way succeeds in ejecting the blood that reaches it from the cardiovascular circulation with greatly reduced effort, and
-- contract, so as to expel the blood collected inside the sac 3 towards the aorta, thus replacing the ventricle of the wearer in the work of pumping the blood towards the vascular system.

In the example illustrated in figures 1 and 2, the alternating movement of expansion and contraction of the sac 3 is commanded through an actuator of the electromechanical type essentially comprising a pusher plate 5 capable of selectively compressing (in practice of squeezing) the sac 3 against an opposed wall of the casing 2 and an electric motor 6 capable of acting on the pusher plate 5 through a screw. The pusher plate 5 is then capable of being recalled back in such a way as to free the sac 3 and enable the sac 3 to expand and fill with blood.

A detailed description with a possible example of embodiment of such a motor and of the members associated to it (and in general of the pumping unit illustrated in figure 1) may be found in patent applications EP-A-0 728 488 and EP-A-0 728 489, mentioned above.

The motor 6 is generally commanded by an electronic control device 8, that for preference can be transported and worn as indicated in figure 2.

Reference number 7 illustrates a further conduit (normally leading to a transcutaneous line, when the device 1 is implanted in the patient's body) whose function is to enable the inflow and outflow of air towards and away from the internal volume of the casing 2 of the device with respect to a volume external with regard to the patient's body. The said gaseous inflow/outflow is evidently induced by the variations in volume to which the sac 3 is subjected by effect of the alternating movement of expansion/contraction commanded by the actuator 5, 6.

The inflow and outflow of air towards and away from the internal volume of the casing 2 of the device with respect to an external volume is important for the purpose of avoiding that inside the casing 2 of the VAD a condition of high pressure comes about that would hinder the sac 3 from filling with blood of and thus the entire functioning of the assistance system. It must normally therefore not be impeded, although sometimes it may be controlled to achieve specific purposes. This is the case when light vacuum is applied to the conduit to favour filling of the sac 3, and thus emptying of the ventricle of the natural heart, or again when the outflow of air from the casing 2 of the VAD is obstructed, progressively and in a highly controlled manner, in order to control the pressure that the heart ventricle must exercise to pump the blood into the sac 3 of the device 1.

An operating principle of this sort, underlying the solutions described in the documents EP-A-1 066 840 and EP-A-1 466 635, the latter being taken as a model for the introductory part of claim 1, is finalised to vary in a controlled manner the degree of assistance provided by the VAD to the natural heart.

The conduit 7 normally terminates with a filter unit 9 necessary to filter the air and avoid entrance, into the inside of the casing 2, of dust that might damage the actuator 5, 6. The conduit 7 may also contain, as in the case illustrated in figure 2, the electric cable providing the connection between the electronic control device 8 and the motor 6. The VAD system is completed by an appropriate power-supply system such as that comprised of rechargeable and wearable batteries 10 indicated in figure 2.

The diagram of the VAD device described makes reference to pulsatile VAD devices with implantable pumps and with actuator that is operated electromechanically. Other models of VAD also exist, known as paracorporeal, in which the pump is situated outside the human body, close to the abdomen, and connected to the heart and to the aorta through two longer conduits 4 that pass through the skin. Again, pulsatile VADs also exist in which compression and release (that is contraction and expansion) of the sac 3 are achieved with different actuation systems: electromechanical (HeartMate system, produced by the company Thoratec in the Unitited States) or electromagnetic (Novacor system, produced by the Canadian company WorldHeart), electrohydraulic (such as the HeartSaver system, developed but not produced by the Canadian company WorldHeart) or pneumatic (such as the pneumatic VAD systems produced by the companies Thoratec in the USA or BerlinHeart or Medos in Germany).

The principal therapeutic goal of ventricular assistance is currently to support the patient's heart for sufficient time to enable its condition to be stabilised and to find a donor heart appropriate for a heart transplant: this procedure is known as "Bridge To Transplant", or BTT. More recently, VADs have also been used, although on a smaller scale, to assist in a permanent fashion patients who, due to their age or to concomitant diseases, cannot be considered as candidates for a transplant. The increasing experience in the use of VADs in the BTT procedure shows that, in some patients, the condition of rest provided to the heart by the VAD is capable of producing a substantial recovery in ventricular function, such as to justify removal of the device without proceeding to a heart transplant. This assistance effect provided by the VAD is frequently known as "Bridge To Recovery".

The percentage of patients in whom it is possible to achieve functional recovery of the heart through assistance with VADs is still relatively low, and varies widely from one centre to another; furthermore, some of the patients remain stable and in good health for years, whereas others are subject to recurrent cardiac insufficiency.

The high variability found in the degree of cardiac recovery achieved with the assistance of a VAD is probably due to different patient conditions, different protocols adopted for patient selection and treatment and, most probably, also to the different characteristics of the devices used and to the different approaches adopted to control such devices.

At present, at the clinical level, numerous different types of VAD are being used to support the circulation in patients at risk of death through non-reversible left ventricular cardiac insufficiency. These VADs have very different characteristics and their operation may be controlled in very different ways.

Usually, continuous flow VADs only partially relieve the left ventricle of the natural heart from its work and, fundamentally, eliminate the natural pulsating nature of the flow and of the pressure in the aorta: however, these factors are significant to guarantee optimal perfusion of the organs and in particular of the heart itself.

Pulsatile flow VADs may be operated either maintaining synchronisation of their phases of filling and emptying with the systolic and diastolic phases of the natural heart but, more frequently, they are controlled at a different frequency from that of the cardiac rhythm.

When the VAD is operated at the same frequency as the heart beat and the relation of phase between the two cycles is controlled in such a manner that the VAD is in the filling phase during the systolic (ejection) phase of the heart and on the contrary is in the ejection phase during the diastolic (filling) phase of the heart it is said that the VAD is operating in "synchronous counterpulsation" with regard to the heart.

It has long been known (see for example S.R. Igo et al. "THEORETICAL DESIGN CONSIDERATIONS AND PHYSIOLOGIC PERFORMANCE CRITERIA FOR AN IMPROVED INTRACORPORAL (ABDOMINAL) ELECTRICALLY ACTUATED LONG-TERM LEFT VENTRICULAR ASSIST DEVICE ("E-TYPE" ALVAD) OR PARTIAL ARTIFICIAL HEART"; Cardiovascular Diseases, Bulletin of the Texas Heart Institute; 1978. Vol. 5, Number 2 page 172-186) that the use of VADs operating in synchronous counterpulsation provides numerous advantages, in particular for the purpose of achieving optimal conditions for the recovery of cardiac function. In these conditions, indeed, a maximum decompression regime, or unloading, of the left ventricle of the heart is obtained such that the heart is enabled to operate in stable conditions with regard to the beat.

On the contrary, if the VAD is operated without maintaining synchronism with the heart beat, the left ventricle finds itself working in highly variable pressure conditions from one beat to the next, depending on the random relationship of phase between the cycles of the VAD and the cardiac cycles.

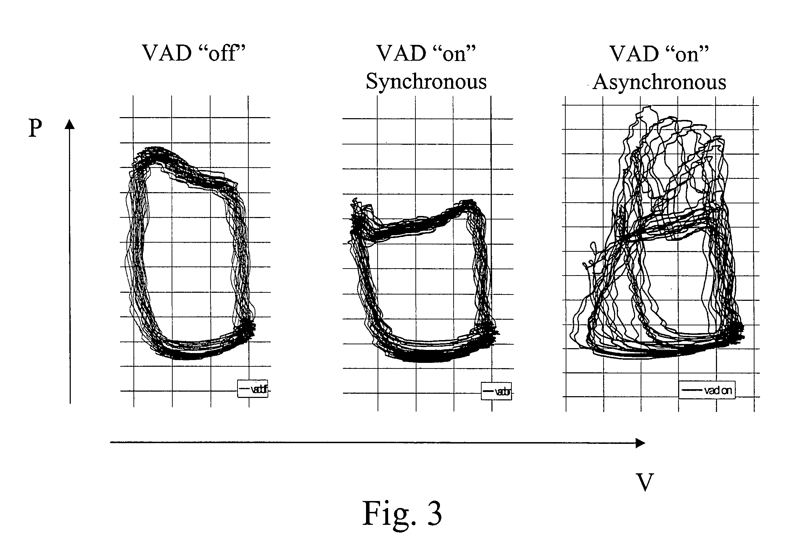

This is for example illustrated in figure 3, in which the pressure-volume curves, or p-v loops, are reported as they are measured inside the left ventricle during an in vivo experiment (on a calf) during which the implanted VAD was operated in different modalities: Off (left-hand curve), On, in synchronous counterpulsatian (central curve) or operated in asynchronous modality (right-hand curve).

The principal disadvantage deriving from operating VADs in synchronous counterpulsatian derives from the fact that the heart is relieved of its work to such an extent that it might undergo progressive atrophy, similar to what occurs with the skeletal muscles of limbs immobilised for long periods, as for example comes about when the limb is put in plaster due to a fracture.

It is however believed that the consequences of this process may be avoided if an appropriate "weaning phase" is applied, before proceeding to removal of the VAD at functional recovery of the myocardium; during this weaning phase the heart is progressively trained to take up its work again. This may be achieved by adopting appropriate clinical protocols such as those suggested by R. Hetzer et al. ("Bridging-to-recovery"; Ann Thorac Surg 2001;71:S109-S113) or by J.K.F Hon et al. ("Bridge to recovery with the use of left ventricular assist device and clenbuterol"; Ann Thorac Surg 2003;75:536-S41) or using appropriate accessories such as those described in EP-A-1 066 840 and EP-A-1 466 635, mentioned above, or again by systems such as that proposed in US-A-4 756 302.

Operation of the VAD in synchronous counterpulsation with respect to the heart beat requires the heart beat itself to be detected. This detection may be achieved by measuring the heart's electrical activity through appropriate electrodes (electrocardiogram, ECG) and processing this signal through known techniques to generate a signal that is appropriate to trigger the operating cycle of the VAD in order to time it. This solution is used in the electronic control units of some VADs (for example the pneumatic paracorporeal system produced by Thoratec in the USA). However, it requires the application of ECG electrodes to the patient, which are not always stable, and the use of additional instruments, not easily transportable, that further reduce the patient's mobility and quality of life.

A further method to detect cardiac activity and synchronise the operation of the VAD with it consists in detecting, with appropriate sensors, the filling with blood of the deformable sac 3. This filling is indeed normally produced by the ejection of blood by the left ventricle during its systole. The blood flow entering the sac 3 has thus, at each cardiac cycle, a peak that starts at the beginning of the heart's systole and terminates at the end of the systole. Analysis of this signal, through known techniques, again enables an adequate trigger signal to be generated to time the operating cycle of the VAD in synchronous counterpulsation with the natural heart. This solution is adopted for example in the VAD system "Novacor" produced by the company WorldHeart (Canada) and described, for example, by P.M. Portner et al. in "A Totally Implantable Ventricular Assist Device For End Stage Heart Disease" in Assisted Circulation 2, Edited by F. Unger, Springer Verlag Berlin, 1984, page 115-141. A similar solution for prototypes of the system HeartMate (now produced by the company Thoratec - USA) is also described by W.F. Bernhard et al. in "Development of pneumatic and electrical VADs with textured blood contacting surfaces for use in temporary and permanent Left Ventricular Bypass", Chapter 3 of ASAIO Primers in artificial organs, 1987, J.P. Lippincott CO (Philadelphia).

Both of these methods require the use of specially-constructed transducers and/or sensors integrated into the blood pump. This involves an increase in the complexity, weight and dimensions of the most critical component of VAD systems, the implantable blood pump. This part of the VAD system, and thus also the means necessary to detect the heart beat and thus maintain synchronous counterpulsation, is normally implanted and is in any case connected to the cardiovascular system. It cannot be replaced or subjected to maintenance or overhaul except through complex and risky surgical operations. Therefore these solutions are not optimal and are highly critical, particularly in terms of the reliability and duration of the system.

### Object and summary of the invention

The description of the related art provided above shows that, despite the relatively large number of proposed solutions, a need is still clearly felt to have available a perfected solution able to overcome the drawbacks described above. Specifically, the need is felt to have available a solution that enables operation of the VAD to be controlled in a synchronous manner in counterpulsation with the natural heart without employing electrodes applied to the patient nor additional instruments and, at the same time, without adding complexity, weight or volume to the implantable pump.

The present invention has as its object that of providing such a perfected solution.

According to the present invention, this object is achieved thanks to a device having the characteristics described in the attached claims. Functioning of the VAD device according to the present invention is controlled by an electronic controlling device containing a programmable micro-processor (controller). The invention thus also concerns a computer programme product that can be loaded into the memory of an electronic computer such as a programmable controller and comprising software code portions that, when the product is run on a computer, causes the computer to operate the pulsatile VAD according to criteria underlying the invention, for example in a synchronous manner in counterpulsation with regard to the natural heart. This purpose is achieved without employing electrodes applied to the patient, nor additional instruments and, at the same time, without adding complexity, weight or volume to the implantable pump.

The claims form an integral part of the disclosure provided here in regard to the invention.

In brief, according to the embodiment at present preferred, the solution described here is based on detecting the gaseous flow (usually air) that moves through a percutaneous line due to variations in volume of the flexible sac of the VAD that, cyclically, fills and empties. For preference, attention is focussed on the discharged air, that corresponds to filling of the VAD and thus to ejection from the natural heart (systole). Measurement of this flow enables the cardiac systole to be the detected and thus enables the VAD to be synchronised to the natural heart. In particular, it is thus possible to operate in the modality known as "synchronous counterpulsation". This corresponds to having the VAD free to receive blood from the heart when it contracts (systole) and having ejection from the VAD to the aorta when the heart is filling (diastole).

The solution described here employs a system to detect the heart beat external to the blood pump, easily replaceable or repairable, without any contact (electric or other) with the patient and directly connectable to the electronic control unit of the VAD without the use of external instruments.

### Brief description of the attached drawings

The invention will now be described, as a simple example and without limiting intent, with reference to the attached drawings, in which:
-- figures 1-3 have already been described,
-- figure 4 illustrates, in diagram form, with direct reference to the diagram in figure 1, a possible embodiment of the solution described here,
-- figure 5 is a time chart representative of the functioning of the solution described here, and
-- figure 6 comprises two time charts that are further representative of the functioning of the solution described here.

### Detailed description of examples of embodiments of the invention

Although the present description makes reference to a VAD with electromechanical operation, those of skill in the art will immediately appreciate that the invention applies in general also to VADs with different actuation systems, such as those to which reference was made in the introductory part of this description.

Indeed, the present invention principally concentrates on the alternating gaseous flow that occurs in the conduit 7, which -- as was described above -- is produced, and thus is caused, by the contraction and expansion movements of the pumping sac, regardless of the ways and means adopted to produce the contraction movement and enable expansion of the variable-volume chamber comprising the sac 3.

The proposed solution, similarly to known solutions developed for the systems Novacor and HeartMate described above, is based on detecting the filling of the blood sac 3 of the VAD. This detection is not however achieved directly through sensors applied inside the blood pump, on the sac itself or on the actuator, but rather indirectly by measuring the flow of air coming out, through the conduit 7, from the casing 2 of the VAD to compensate for the reduction in free volume inside it caused by the filling of the sac with blood.

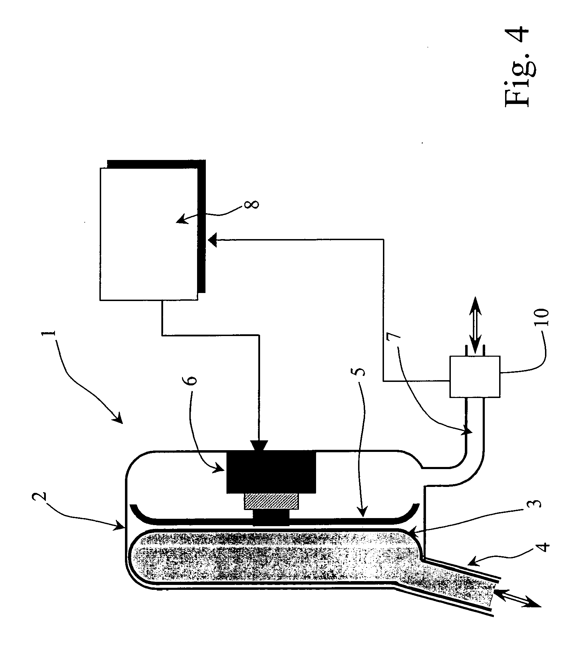

As is represented in diagram form in figure 4 (where parts and elements equal or equivalent to those already described with reference to figure 1 are indicated with the same numbers) measurement of said flow of air is achieved through a sensor 10 associated to the conduit 7.

The sensor 10 is thus capable of generating a signal that is indicative of the flow in the conduit 7 and thus of the cyclic inflow and expulsion of blood into and out of the sac/variable-volume chamber, that is in practice, of the functioning of the VAD as a pumping device. In particular, the gaseous flow leaving the conduit 7 is indicative of the filling of the sac 3 of the VAD and thus of the ejection of blood from the left ventricle of the heart, that is of its systole.

Typically, the sensor 10 generates a signal indicative of the rate of flow (volume per unit time) or of the speed of flow of the gaseous matter in the conduit 7 and consequently indicative of the flow of blood ejected from the left ventricle of the heart towards the flexible sac 3 of the VAD.

The sensor 10 may consist of a flow meter of known type, such as a flow meter for gases or a device comprising a pressure transducer (for example piezoelectric) situated upstream of a constriction or fluid resistance.

For example, the flow meter 10, whose output signal is provided to the electronic unit (controller) 8 that supervises operation of the VAD 1 overall, may to advantage consist of the flow meter produced by the company Honeywell and available on the market under the code number AWM43600V. It may if desired be mounted in parallel to a small bypass conduit useful to adapt the interval of flow characteristic of the commercial flow meter to that of interest for the specific application (for example between 0 and 40 litres/minute).

It will be appreciated that the sensor 10 lends itself to being situated external to the blood pump (connected to the circulatory system and if desired implantable inside the human body), associating it in very diverse manners, for example introducing the sensor 10 into the conduit 7 or integrating it into the filter unit 9 (figure 2). Again, the sensor 10 might comprise one or more probes associated to the conduit 7 to detect the gaseous flow through it with a sensor module proper situated in a remote position with regard to the conduit 7: it is thus clear that the modalities and criteria to "associate" or "couple" the sensor 10 to the conduit 7 may be of the most diverse.

In any case, the most critical part of the VAD system, the pump, is not needlessly made more complex by the addition of the sensor or sensors needed for its operation in the "synchronous counterpulsation" modality and at the same time these sensors, here represented by the sensor 10, are situated in positions easily reachable for any replacement or repair action.

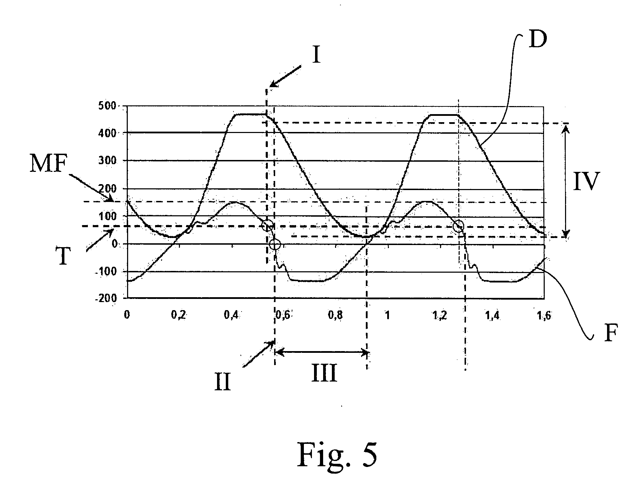

Measurement of the flow of air leaving the conduit 7, see curve F in figure 5, confirms that its time trend closely reproduces from the qualitative standpoint the trend of the flow of blood filling the sac 3 of the VAD.

This signal may be acquired and processed with simple techniques, of themselves known, by the electronic control unit 8, which is therefore capable of commanding the pumping unit 3-6 in function of the signal generated by the sensor 10. Just as it is incidentally clear to technicians who are experts in the sector, that in stating that the electronically controlled unit 8 is capable of commanding the pumping unit 3-6 as a function of the signal generated by the sensor 10 it is not in any way intended to imply that control of the pumping unit is only carried out as a function of the signal of the sensor 10: in reality, the unit 8 receives the flow signal from the sensor 10 and utilises that signal to control the functioning of the pumping unit 3-6, together with other parameters/signals available to the unit 8. For example, in the case of actuators operated by an electric motor, the electronic control unit 8 generally has available to it signals relating to the motion of the motor 6 and/or of the pusher plate 5 provided by specific encoders.

This situation may easily be verified in a VAD 1 of the type described here by observing the fact that, other conditions being equal, the device behaves in a different way, that is the unit 8 commands the pumping unit 3-6 in a different manner, depending upon whether the sensor 10 is connected or is not connected to the unit 8.

For example, the unit 8 may detect the maximum MF in the curve of the flow leaving the conduit 7 and wait for the flow to drop below a threshold T, defined as a given percentage fraction of the maximum flow MF, substantially corresponding to the end of the filling peak of the sac 3 and thus to the end of the systole of the natural heart. The electronic control unit 8 may at that instant command the actuator 6 such as to start a cycle of movement of the pressure plate 5, such as that illustrated in figure 5 by curve D: compression of the sac (ejection of blood from the VAD to the aorta) and return to the retracted position (sac free to fill again) in expectation of the start of the subsequent cycle.

It must be stressed that, in general, analysis of the flow signal may be performed on the basis of the shape of said signal without the need to have available absolute values of the gaseous flow measured. This leads to intrinsic system safety since it makes the control system independent of any variations in sensitivity of the flow meter consequent on changes in temperature or on ageing.

For further reference, the scale of the abscissa in figure 5 is a time scale quoted in seconds in which, as well as the values already mentioned above, the following are indicated:
-- with I, the instant at which the flow drops below the threshold T defined above and at which the control unit 8 commands the start of the movement of the pusher plate 5 aimed at producing contraction of the sac 3,
-- with II, the instant at which the VAD begins ejection (crossover point of zero flow in the conduit detected by the sensor 10),
-- with III, the interval of ejection of the VAD, and
-- with IV, the amplitude of the movement squeezing the sac 3 by the pusher plate 5 (representative in practice of the charge of blood delivered) after contact with the sac 3.

It will be appreciated that the instants I and II do not coincide. Indeed, it is usual to operate such that (according to known criteria) in the release movement of the sac 3 that leads the sac 3 to fill with blood, the pusher plate 5 disengages from the wall of the sac in order to leave the sac 3 completely free to expand and receive blood. In consequence, in commanding contraction of the sac, the pusher plate 5 must initially make a brief movement to regain contact with the sac 3 and to begin ejection. From observation of this fact it appears that, in an independent manner from any other consideration, the action of monitoring the gaseous flow in the conduit (percutaneous line) 7, achieved through the sensor 10, also makes it possible to detect the movement of expulsion of the blood with regard to the sac 3 in a much more precise and faithful fashion than could be done, for example, by monitoring movement of the pusher plate 5. This precisely because the gaseous flow in the conduit 7 is caused by the contraction/expansion of the sac 3 which acts as a flexible pumping chamber for the blood.

Other control logics are naturally possible; it is for example possible to detect the start of the systole of the natural ventricle in correspondence with the maximum slope of the rising part of the air flow leaving the conduit 7, and control operation of the VAD starting from detection of this instant, which is closely correlated, in the cardiac cycle, to the start of the systole.

The signal corresponding to the air flow leaving the conduit 7 is appropriate to be acquired and processed by the electronic unit 8 of the VAD in such a manner as to control operation of the VAD in a synchronous manner and in counterpulsation with the heart. This result conforms to what is illustrated in figure 3 (central curve: VAD On - synchronous). In particular, the above-mentioned signal of cardiac rhythm indicative of the systolic and diastolic phases of the heart C assisted by the device 1 is provided by the sensor 10 sensitive to the gaseous flow in the conduit 7.

In programming the electronic unit 8 that controls the VAD, as was said above, an operational cycle is defined that comprises, for example:
-- compression of the sac (ejection of blood by the VAD),
-- return to the retracted position (sac free to fill again),
-- wait to detect the end of a subsequent cardiac systole before starting the next cycle.

In programming the electronic unit 8, a maximum value may be set for the above wait time. In this case, should no systole be detected, due to a period of cardiac asystolia or a malfunctioning of the flow sensor 10, the VAD 1 is activated at a fixed frequency. This ensures that the system operates in a condition of safety, without allowing cardiocirculatory assistance to lack, either in the case of an irregular cardiac cycle or in the case of malfunctioning of the cycle detection system. Should the cause of a failure to detect the cardiac systole be an arrhythmia (such as for example an asystolia) followed by a return to a regular cardiac rhythm, the system to detect and process the air flow leaving the conduit 7 enables the control unit 8 to become aware of the return to a regular rhythm and to re-synchronise operation of the VAD with the heart beat.

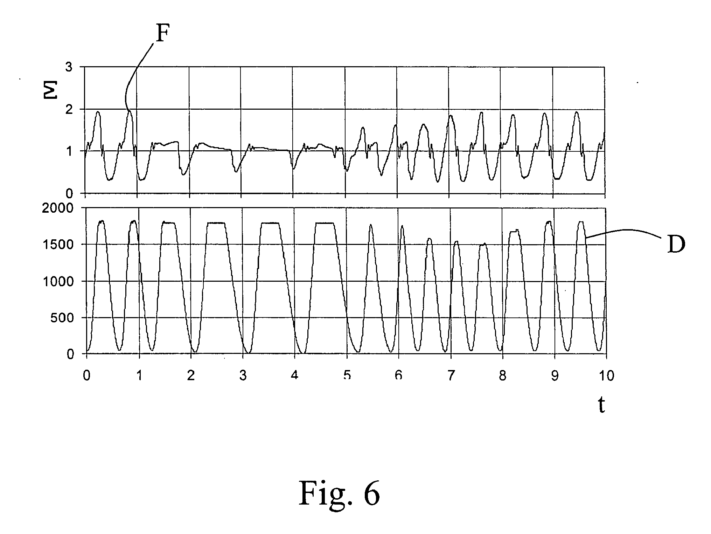

This is illustrated in figure 6, in which in the upper curve a possible trend is shown as a function of time (scale on the abscissa, in seconds) of the air flow signal (here, too, indicated with F, ordinate scale, indicated in arbitrary units). In the lower curve, on the contrary, a possible co-ordinated time trend is shown (same scale on the abscissa) of movement of the pusher plate 5 (here too indicated with D, ordinate scale, in hundredths of a millimetre) of the VAD 1 commanded by the electronic control unit 8 in the case of an asystolia of the duration of approximately 5 heart beats.

It may be noted that, during the asystolia, the VAD is activated at a fixed frequency (movement of the pusher plate with long and constant wait times) whereas on return to the regular cardiac rhythm, operation of the VAD, during a small number of cycles, returns to being synchronous and in counterpulsation.

Naturally, without prejudice to the underlying principles of the invention, construction details and embodiments may vary, even significantly, with regard to what is described here as a simple example without limiting intent, without thereby departing from the scope of the invention, as defined by the attached claims.

## Claims

1. A cardiac ventricular assistance device (1) comprising:
- a pumping unit (3-6) with a variable-volume chamber (3) situated inside a casing (2) and capable of receiving a mass of blood,
- a control unit (8) to obtain, through said pumping unit (3-6):
i) contraction of said variable-volume chamber (3), with consequent expulsion of the blood collected in the chamber (3) and
ii) expansion of said variable-volume chamber (3), with consequent inflow of blood into the chamber (3), and
- a gaseous flow line (7) to permit gaseous inflow and outflow with regard to said casing (2) as a result of contraction and expansion of said variable-volume chamber (3),
**characterised in that** :
- a sensor (10) is provided sensitive to the gaseous flow in said flow line (7) to generate a flow-meter signal that, being indicative of the gaseous flow in said flow line (7), is indicative of the inflow and expulsion of blood with regard to said variable-volume chamber (3), and
- said control unit (8) is sensitive to said flow-meter signal and utilises said flow-meter signal to control said pumping unit (3-6).

2. Device according to claim 1, **characterised in that** said gaseous flow line (7) is a transcutaneous line.

3. Device according to claim 1 or claim 2, **characterised in that** said sensor (10) is situated along or within said flow line (7).

4. Device according to claim 1 or claim 2, **characterised in that** said flow line (7) includes or terminates in a filter unit (9) and **in that** said sensor (10) is situated in said filter unit (9).

5. Device according to any of the above claims, **characterised in that** said control unit (8):
- is also sensitive to a cardiac rhythm signal indicative of the systolic and diastolic phases of the heart (C) assisted by the device (1),
- is configured to control said pumping unit (3-6) in a condition of synchronous counterpulsation, with said variable-volume chamber (3) capable of receiving blood from said assisted heart (C) when said assisted heart is in systolic phase and with said variable-volume chamber (3) that expels the blood collected in the chamber (3) when said assisted heart (C) is in diastolic phase.

6. Device according to claim 5, **characterised in that** said cardiac rhythm signal indicative of the systolic and diastolic phases of the heart (C) assisted by the device (1) is achieved by said flow-meter signal generated by said sensor (10) sensitive to the gaseous flow in said flow line (7).

7. Device according to any of the above claims, **characterised in that** said control unit (8) is configured to detect the passage of said flow-meter signal below a threshold (T) indicative of the end of blood flow into said variable-volume chamber (3) and at said passage to command the start of a cycle comprising the contraction of said variable-volume chamber (3), with consequent expulsion of the blood collected in the chamber (3), and the expansion of the variable-volume chamber (3), with consequent inflow of blood into the chamber (3).

8. Device according to claim 7, **characterised in that** said control unit (8) is configured to determine said threshold (T) as a given fraction of the maximum flow (MF) of said flow-meter signal.

9. Device according to any of the above claims, **characterised in that** said control unit (8) is configured to detect the start of the systole of the heart (C) assisted by the device (1) in correspondence with the maximum slope of the rising part of said flow-meter signal and control operation of the device (1) starting from the beginning of the systole thus detected.

10. Device according to any of the above claims, **characterised in that** said control unit (8) is configured to control the device (1) according to cycles, comprising:
- contraction of said variable-volume chamber (3), with consequent expulsion of the blood collected in the chamber (3),
- expansion of said variable-volume chamber (3), with consequence inflow of blood into the chamber (3), and
- wait to detect the end of a subsequent systolic phase of the heart (C) assisted by the device (1) before starting the successive cycles.

11. Device according to claim 10, **characterised in that** said control unit (8) is configured to set a maximum value for said wait time to detect the end or the start of a successive systole of the heart (C) assisted by the device (1).

12. Device according to any of the above claims, **characterised in that** said control unit (8) is configured to control said pumping unit (3-6) with fixed pumping frequency in the presence of an event chosen from among:
- failure to detect a systole of the heart (C) assisted by the device (1), and
- absence of said flow-meter signal.

13. A computer program product loadable into the memory of computer and including software code portions that, when the product is run on a computer, commands the operation of said computer in such a way that said computer acts as said control unit (8) of the device according to any of the claims from 1 to 12.
